# EUROPEAN PATENT APPLICATION

(11) **EP 4 571 624 A1**
(43) Date of publication of application: **18.06.2025**
(21) Application number: 23853068.7
(22) Date of filing: 11.08.2023
(51) Int. Cl.: G06Q 30/06, G06Q 30/02, G06F 16/29, G06F 16/909, G06F 16/9537, G06T 19/00

(54) **META-SPACE-TIME PRODUCT TRADING DEVICE AND METHOD BASED ON META-SPACE-TIME PRODUCT COORDINATE GENERATOR, AND META-SPACE-TIME PRODUCT SEARCH AND ACCESS DEVICE**

(30) Priority: 11.08.2022 KR 20220100989
(71) Applicant: Boom and Dream Vacation Corp., Daejeon 34071 (KR)
(72) Inventor: KIM, Ki Joung, Daejeon 34075 (KR); LEE, Woo Sung, Hanam-si Gyeonggi-do 12912 (KR); JEONG, Jong Tae, Daejeon 35333 (KR); KIM, Min Jae, Daegu 41418 (KR); PARK, Seo Jun, Daejeon 35367 (KR)
(74) Representative: Budde Schou A/S
(86) International application number: PCT/KR2023/011929
(87) International publication number: WO 2024/035202

(57) **Abstract**

Provided is a meta-space-time product trading device based on a meta-space-time product coordinate generator. The device is a meta-space-time-based product trading device that transcends time and space, and comprises: a meta-space-time product coordinate generator which generates meta-space-time product coordinate information for a specific product in which coordinates corresponding to specific latitude information, longitude information, and height information are integrated into a single set of coordinates, and assigns the meta-space-time product coordinate information to the specific product; and a resale product information database which is linked to the meta-space-time product coordinate generator and in which information about the product assigned the meta-space-time product coordinate information is recorded. In addition, the device may comprise a purchase interface, which is linked to the resale product information database, for trading the meta-space-time product coordinate information or receiving, via the Internet, data for trading the product assigned the meta-space-time product coordinate information.

## Description

### [Technical Field]

The present invention relates to a device and method for identifying or detecting in real time an actual location and height of a product that is being sold online or offline, generating meta-space-time coordinates in which time (or period) information is integrated into the detected information, and assigning the generated meta-space coordinates to the corresponding products to search for and access the product online according to the meta-space-time coordinates and trade the product, and more particularly, to a device and method for identifying or detecting a specific product in real time (through artificial intelligence, etc.) through image information, virtual reality (VR), augmented reality (AR), a barcode, sound, smell, pressure, specific code information, etc., and assigning the meta-space-time coordinates to the identified or detected product to search for and access the product assigned the meta-space coordinates online, thereby allowing netizens, who are third parties, rather than an original seller of the product, to trade the products and sharing a certain amount of revenue with the corresponding netizens.

### [Background Art]

As well as being the reason why the current Internet exists, the fundamental system that constitutes the Internet is data search. This means that it is as if data that does not need to be searched by anyone or data that cannot be retrieved did not exist.

The Internet is a computer network communication network that is connected worldwide based on the Internet protocol suite (transmission control protocol/Internet protocol (TCP/IP)). Since a search is based on the existence of data, a search in the existing Internet system has a structure in which, when a user inputs schedule information on a web page as search terms, a server that stores the web page related to the schedule information responds to the search terms and allows the user to view the corresponding web page or related information of result values on a screen via the Internet network.

In the meantime, countless different Internet services have been launched worldwide. In these services, the existing search method and the advertising market are directly connected, which means that users' search terms are used for commercial purposes.

In other terms, as the number of users has increased exponentially, the number of search terms input by users has also increased dramatically, and therefore search terms input by users are used as a means for large portal sites with large web pages to benefit economically.

There are many different types of advertising products related to search terms. These advertising products may mainly display advertisements when search terms related to users' personal information (various search terms input by users, actual locations of user terminals, etc.) are entered from the user terminals or when there is a search using search functions related to the personal information of users (matching or including, mismatching, not including, etc., personal information).

As an example of the existing advertisements using the personal information, there may be a case where a user A searches the term "fan" on a search site to find out the price of a fan, and then banner advertisements that allow the user A to purchase fans at each site that the user A visits appear on the user A's screen.

As another example of the existing advertising, similarly, there may be a case where, when a user B searches posts (or products) related to the term "fan" to find out the price of a fan, posts with advertisements related to fans first appear at the top of a page where the search results appear, or banner advertisements related to fans appear at a certain location on the page.

In this way, existing online advertisements have generally used a method of displaying advertisements on screens of user terminals where search terms related to search terms set by advertisers are input to dispose advertisements or use related search functions (matching, including, mismatching, not including, etc., personal information), or when actual user terminals are at locations set in advance by advertisers to dispose advertisements, displaying the advertisements on the corresponding terminals to induce purchase.

Recently, Apple has introduced "app tracking transparency" as a privacy policy. Conventionally, companies such as Facebook have been selling advertising products that may be displayed as targeted advertisements by tracking and collecting large portions of users' personal information (search terms that users search, actual location information of user terminals, etc.). However, according to Apple's policy, an era has arrived in which advertisers cannot track personal information of users using Apple devices without the consent of the users.

In other terms, an era has arrived in which technologies that enable data search on the Internet by new methods are needed, rather than the existing methods where personal information is the main search medium.

Recently, in the case of products or services sold in online shopping malls among various services for e-commerce, the lowest selling price is mostly disclosed through major portal sites, and the information on the lowest selling price is provided to people as a universal service so that anyone can know the lowest selling price.

Due to this environment, competition between companies is becoming increasingly fierce, and naturally, even if advertising expenses are spent, a margin due to advertising expenses is decreasing.

In recent years, most online e-commerce sales methods have involved having product sellers spend money and time to take photos of products to be sold one by one, edit the photos, and upload the photos onto a server on the Internet along with related information, etc., after which the advertised products are purchased from portals with large numbers of web pages and visitors, etc., and corresponding advertisements are displayed to induce users to purchase the products.

Recently, various online platforms have been launched. For example, Amazon in the US has opened its database and services to the outside in the form of an open application program interface (API). This allows other websites to pick up and upload information such as prices and product details from Amazon's product database (DB) and use Amazon's payment system and shopping cart.

This means that an era has arrived in which even ordinary netizens who do not actually have products can benefit economically from activities that affect online sales on the Interne.

As discussed above, since it is becoming increasingly difficult for advertisers to secure users' personal information (such as search terms input by users or actual location information of user terminals) as a means of displaying advertisements, a new search method is required, and a new product trading method is required accordingly.

In addition, the existing technologies can be used to search for products related to time information by adding the time information as a search term, but there is a problem that the existing technology can only serve to search for products related to tag information containing information on time instead of search terms, as in the case of using general search terms.

In addition, when users want to search for a product that actually corresponds to space coordinates corresponding to a specific height from a location specified by a specific latitude and longitude, even if the corresponding space coordinates are only one specific set of space coordinates, the corresponding space coordinates are all different space coordinates, which exist almost infinitely, depending on past, present, and future points in time, and a technology capable of mainly searching for and trading products according to the space-time coordinates into which countless time coordinates are integrated is required.

The existing related technologies are generally used to provide people with information on an address of a store, a price of a product, etc., to sell the product or service, and do not allow products with integrated space coordinates and time coordinates according to all the space coordinates of a boundary of an area occupied by the corresponding store within a building or the specific latitude, longitude, and height information for the products displayed within the store to be easily searched for. In addition, even if there is only one specific product, when the specific product is related to countless space-time coordinates, it is impossible to distinguish transaction conditions of the same product and trade the product according to each existing set of space-time coordinates.

However, as in the case of Amazon described above, an era has arrived in which, even if netizens do not actually have a product (or have a service), online technology capable of reselling products (or services) that are already being sold is required. For example, an overseas tourist finds a pair of shoes he/she likes at a height of 1 meter on a left shelf of a shoe store on the third floor of a shopping center while traveling. Thereafter, when the tourist wants to show the shoes to other people and encourage people to purchase the shoes for the purpose of reselling the shoes, a technology capable of allowing people to search for a product at space coordinates corresponding to the specific height from the location specified by the corresponding latitude and longitude and the products to be resold to netizens who have searched for the products is required.

Meanwhile, in the case where the space-time coordinates of the displayed shoes are likely to change, a technology capable of searching for and trading shoes according to different space-time coordinates that change in such cases is required.

### [Disclosure]

### [Technical Problem]

The present invention is intended to solve the problems of the above-described related art, and there is a problem in that the existing data storage system stored on the Internet does not necessarily assign and store meta-space-time coordinates into which the actual location (latitude and longitude) and height information for a product (or service) that is being sold in an online or offline store, etc., and time (or period) information for a specific coordinate corresponding thereto are integrated.

Accordingly, the present invention is to identify or detect a product (or service) in real time (through artificial intelligence, etc.) through image information, VR, AR, a barcode, sound, smell, pressure, etc., in order to assign space-time coordinates to an online or offline product or service in real time, and to allow netizens, who are third parties, rather than original sellers, to search for and access the product assigned the integrated space-time coordinates online through the space-time coordinates so that the netizens can resell the product, thereby sharing a certain amount of revenue with the corresponding netizens.

### [Technical Solution]

A meta-space-time product trading device for implementing the present invention may be configured to include a meta-space-time product coordinate generator and a purchase interface, a geographic information database, a store information storage device, a product information storage device, a resale product information database, a trading information log database, a transaction data block, etc.

The meta-space-time product coordinate generator may be configured to include a meta-space-time product coordinate generation terminal (hereinafter referred to as a product coordinate terminal), and configured to include an account access interface, a product identification interface, a store setting interface, a detailed product information interface, a meta-space-time product coordinate setting interface, a resale information generation interface, a resale approval interface, a product selection interface, etc.

The account access interface may be configured to include a reseller account login module, an original seller account login module, a buyer account login module, a settlement management module, an account information input module, etc.

The product identification interface may be configured to include a product identification module archive indicator, an image information identification module, a virtual reality (VR) identification module, an augmented reality (AR) identification module, a barcode identification module, a sound detection module, a smell detection module, a pressure detection module, a product identification code information input module, a product location information identification module, a product height information identification module, etc.

The store setting interface may be configured to include a store archive indicator, a store location information identification module, a store selection information identification module, a detailed store information indicator, etc.

The detailed product information interface may be configured to include a detailed product information indicator, etc.

The meta-space-time product coordinate setting interface may be configured to include a product location coordinate setting module, a product height coordinate setting module, a product point-in-time coordinate setting module, a product period coordinate setting module, etc.

The resale information generation interface may be configured to include a resale product information registration module, a resale product block information generation module, a resale product information registration point-in-time information identification module, a resale product information reset application module, etc.

The resale approval interface may be configured to include a resale product information reset approval module, a resale approval module, etc.

The product selection interface may be configured to include a product search information input module, a product archive indicator, a product selection module, etc.

The meta-space-time product search and access device for implementing the present invention may be installed in a product coordinate terminal and can be configured including a meta-space-time product search interface, a meta-space-time product access interface, etc.

According to another embodiment of the present invention, a method of generating meta-space-time product coordinates using an identification or detection method is a method of identifying or detecting a product or service through a schedule identification or detection means installed in a product coordinate terminal, generating the meta-space-time product coordinates to assign the generated meta-space-time product coordinates to the identified product or service. The method includes: logging in to each account login module of at least one of a reseller account, an original seller account, and a buyer account; identifying or detecting a product by at least one of a product identification interface image information identification module, a virtual reality (VR) identification module, an augmented reality (AR) identification module, a barcode identification module, a sound detection module, a smell detection module, a pressure detection module, and a product identification code information input module of a product identification interface; outputting a store list of products identified (or detected) by the product identification interface or a store list identified by a store location information identification module through a store archive indicator; selecting at least one store from among stores in the store list output from the store archive indicator; identifying at least one of latitude and longitude and height information from at least one of a product location coordinate setting module and a product height coordinate setting module; identifying or setting at least one of information on a point in time and a period from at least one of a product point-in-time coordinate setting module and a product period coordinate setting module; approving a resale of a product to which resale product information is assigned by a resale approval module according to a schedule protocol; assigning at least one of information on latitude and longitude, height, point in time, and period identified or set by at least one of a product location coordinate setting module, a product height coordinate setting module, a product point-in-time coordinate setting module, and a product period coordinate setting module to the comprehensive product information stored in a product information storage device; and generating resale product information for recording resale product information in a resale product information database synchronized with comprehensive product information stored in the product information storage device, and recording the generated resale product information in the database.

According to another embodiment of the present invention, a method of generating meta-space-time product coordinates of a selection method is a method of selecting a product or service through a product selection module, generating meta-space-time product coordinates, and assigning the generated meta-space-time product coordinates to the selected product or service. The method includes: logging in to each account login module of at least one of a reseller account, an original seller account, and a buyer account; selecting the product through the product selection module; outputting a store list of products selected through the product selection module through a store archive indicator; identifying or setting at least one of information on a point in time and a period from at least one of a product point-in-time coordinate setting module and a product period coordinate setting module; approving a resale of a product to which resale product information is assigned by a resale approval module according to a schedule protocol; assigning at least one of information on latitude and longitude, height, point in time, and period identified or set by at least one of a product location coordinate setting module, a product height coordinate setting module, a product point-in-time coordinate setting module, and a product period coordinate setting module to the comprehensive product information stored in a product information storage device; and generating resale product information for assigning resale product information to the comprehensive product information stored in a product information storage device, and assigning the generated resale product information to the corresponding comprehensive product information.

According to another embodiment of the present invention, a meta-space-time-based product trading method is a method for trading a product assigned meta space-time coordinates online. The method includes: inputting at least one of information of latitude and longitude, height, point in time, and period of a product to be searched to input information for searching a product assigned resale product information through a meta-space-time product information input module; outputting a list of products assigned the resale product information on a screen of a product coordinate terminal through a meta-space-time product search result archive indicator; accessing a meta-space-time product access module to access comprehensive product information of one of the products among the list of products output on the screen of the product coordinate terminal; executing a function of purchasing the product assigned the resale product information accessed through the meta-space-time product access module; and storing trading-related information for a resale product in a trading information log database.

### [Advantageous Effects]

As described above, according to the present invention, it is possible to assign the coordinates corresponding to the actual latitude and longitude of the product and the actual height information (or floor of the building, etc.) for the product to the product (service) sold in the online or offline store, etc.

According to the present invention, it is possible to assign the space-time coordinates in which the actual latitude and longitude of the product and the actual height information (or floor of the building, etc.) for the product and the specific time (or period) information are integrated to the product (or service) that is being sold in the online or offline store, etc.

According to the present invention, it is possible to identify or detect the product (or service) that is being sold in an online or offline store in real time (through artificial intelligence, etc.) using visual information, VR, AR, a barcode, sound, smell, pressure, specific code information, etc., identify the actual latitude and longitude of the product (or service) identified or detected in real time and the actual height information (or which floor of the building the product is on) in real time, search for and access the assigned product or service online through the space-time coordinates by assigning the space-time coordinates in which information on a certain point in time and period is integrated with the identified information, thereby allowing the third party, rather than the original seller of the product, to resell the product online and sharing a certain amount of revenue with the third party.

### [Best Mode]

A meta-space-time product trading device for implementing the present invention is disclosed.

A meta-space-time product trading device for implementing the present invention may be configured to include a meta-space-time product coordinate generator and a purchase interface, a geographic information database, a store information storage device, a product information storage device, a resale product information database, a trading information log database, a transaction data block, etc.

The meta-space-time product coordinate generator for implementing the present invention may be configured to include a meta-space-time product coordinate generation terminal (hereinafter referred to as a product coordinate terminal). Such a terminal may be implemented in the form of a tablet, a personal computer (PC), a smartphone, etc., in which a browser, etc., which may access a database, an interface, etc., for the present invention through the Internet, is installed.

The meta-space-time product coordinate generation device for implementing the present invention may be configured to include an account access interface, a product identification interface, a store setting interface, a detailed product information interface, a meta-space-time product coordinate setting interface, a resale information generation interface, a resale approval interface, a product selection interface, etc.

The account access interface may be configured to include
- a reseller account login module,
- an original seller account login module,
- a buyer account login module,
   -- a settlement management module (a function of generating and transmitting command information that instructs transfer of a certain commission to a reseller's account in order to share the revenue with the reseller in relation to a sale of a product, etc.), and
   -- an account information input module (including a module, protocol, etc., capable of accessing a financial server, etc., that stores users' account information or store account information, etc.).

The product identification interface may be configured to include
- a product identification module archive indicator (a function of selecting the identification module below, etc.),
   -- an image information identification module (identification of a product through photos, etc.),
   -- a virtual reality (VR) identification module (identification of a product through VR),
   -- an augmented reality (AR) identification module (identification of a product through AR),
   -- a barcode identification module (identification of a product through 1-dimensional (1D), 2-dimensional (2D), or quick response (QR) code, data matrix code, etc.),
   -- a sound detection module (identification of a product through sound detection),
   -- a smell detection module (identification of a product through smell detection),
   -- a pressure detection module (identification of a product through detection of Braille, pressure, etc.),
- a product identification code information input module (identification of a product through input of a number, figure, special character, password, uniform resource locator (URL), Internet protocol (IP) address, etc.),
- a product location information identification module (a function of transmitting and receiving schedule information by a geographic information database and a schedule protocol to identify latitude and longitude of the product coordinate terminal at the time of identification of product through the identification module. etc.), and
- a product height information identification module (a function of identifying height information of the product coordinate terminal at the time of identification of product through the identification module, etc.).

The store setting interface may be configured to include
- a store archive indicator (a function of displaying a list of stores selling the identified product on the screen of the product coordinate terminal when the product is identified through the module selected in the product identification interface or allowing the user to select a store through the displayed screen, etc.),
   -- a store location information identification module (a function of displaying store information on a screen of the same terminal when the actual location information of the product coordinate terminal accessed by a user or information of a store including or associated with location information according to a certain standard is in a store information storage device, etc.),
   -- a store selection information identification module (a function of recognizing a store selling a product selected through a product selection module, etc.), and
   -- a detailed store information indicator (a function of outputting details for a store selected through the store archive indicator, etc.).

The detailed product information interface may be configured to include (e.g., a function of outputting information such as a photo, a price, a sales time, a sales period, and a resale commission of a product identified in the product identification interface or selected in the product selection interface)
- a detailed product information indicator, etc.

The meta-space-time product coordinate setting interface may be configured to include
- a product location coordinate setting module (e.g., function of setting the latitude and longitude of the product identified in the product identification interface or selected in the product selection interface),
- a product height coordinate setting module (e.g., a function of setting information such as a height from the ground or a height from sea level of the product identified in the product identification interface or selected in the product selection interface, or which floor of a building the product is on, etc.),
- a product point-in-time coordinate setting module (a function of identifying the point-in-time information identified through the product identification interface, of the product identified in the product identification interface or selected in the product selection interface, or setting certain point-in-time information, etc.), and
   *The point in time is a specific date (year, month, day, etc.) and time (hour, minute, second, etc.).
- a product period coordinate setting module (a function of setting period information for the product identified in the product identification interface or selected in the product selection interface, etc.).

The resale information creation interface may be configured to include
- a resale product information registration module (a function of recording resale product information for resetting the product identified through the product identification interface or selected through the product selection interface as a resale product in the resale product information database synchronized with comprehensive product information of the product information storage device, etc.),
- a resale product block information generation module (a function of identifying schedule request information from the resale product information registration module, and generating a URL or IP address or a blockchain or NFT, etc., that may settle a commission related to resale, transfer a certain amount, include or access related information, etc.),
   * "NFT" means "non-fungible token."
- a resale product information registration point-in-time information identification module (a function of identifying the point in time when the resale product information is recorded in the resale product information database synchronized with the comprehensive product information of the product information storage device by the resale product information registration module, and identifying the point in time when the request information for recording the corresponding information occurs or the point in time when the recording of the corresponding information starts or is completed, etc.), and
- a resale product information resetting request module (a function of viewing "resale product information resetting request information" for setting a start or end date of a sales point in time of a product, setting a sales period, resetting a sales price, commission, etc., in an original seller account), etc.

The resale approval interface may be configured to include
- a resale product information reset approval module (a function of changing, approving, or rejecting resale condition information of the product corresponding to the resale product information resetting request information, etc.), and
- a resale approval module (e.g., a function of generating and approving resale approval information according to a schedule protocol for automatic or manual operation, etc.).

The product selection interface may be configured to include
- a product search information input module (product search function),
- a product archive indicator (a function of outputting a list of products in the product information storage device on the screen of the product coordinate terminal, etc.), and
- a product selection module (a function of selecting at least one from the list of products output on the screen of the product coordinate terminal through the product archive indicator, etc.) etc.

The purchase interface for implementing the present invention may be configured to include
- a detailed resale product information indicator (a function of outputting the detailed information of the product corresponding to the comprehensive product information of the product information storage device on the screen of the product coordinate terminal, etc.), and
- a resale product purchase module (a product purchase function output on the screen of the product coordinate terminal, etc., a function of storing trading record information in the trading information log database, etc.).

The geographic information database for implementing the present invention may be configured to include a product location information identification module for storing the latitude and longitude information and identifying the latitude and longitude of the product coordinate terminal at the time of the product identification through the product identification module archive indicator, and a function of transmitting and receiving the schedule information by the schedule protocol, etc.

The store information storage device for implementing the present invention may be configured to include a function of storing comprehensive information for a store, such as a name, a photo, and a location of the store, etc.

The product information storage device (a function of storing comprehensive product information for sales, such as a name, a photo, a price, and a resale commission of a product, etc.) may be configured to include
- a meta-space-time product search request information reception module, etc.

The resale product information database for implementing the present invention may be configured to include the product information storage device, a function of recording related information of the product including the resale product information or the resale approval information, synchronized by the comprehensive product information and the schedule protocol, etc.

The trading information log database for implementing the present invention may be configured to include a function of storing the trading record information, etc.

The transaction data block for implementing the present invention may be configured to include a function of recording certain tag information or hash, proof of work, version, previous block hash, Merkle root, time, difficulty target, mining information, nonce, header, body, information related to a transaction, and related trading record information, etc., in the form of a blockchain, an NFT, etc., for identifying or accessing a certain commission amount or commission rate information for resale, an original seller account, a reseller account, account information, comprehensive product information (latitude, longitude, height, point in time, period, etc.).

The meta-space-time product search and access device for implementing the present invention may be installed in a product coordinate terminal and may be configured to include a meta-space-time product search interface, a meta-space-time product access interface, etc.
- The meta-space-time product search interface may be configured to include
   -- a meta-space-time product information input module (a function of inputting latitude and longitude, height information, point-in-time information, period information, etc., for the product),
   -- a meta-space-time product radius information input module (a function of inputting a radius range from the latitude and longitude of the product, etc.),
   -- a meta-space-time product identification information transmission module (a function of identifying the product related or unrelated to the latitude and longitude, the height information, and the radius information input from the "meta-space-time product information input module" or the "meta-space-time product radius information input module," a function of transmitting the command information instructing transmission of the search result value corresponding to the "meta-space-time product search result archive indicator" to the "meta-space-time product search request information reception module" of the product information storage device, etc.), and
   -- a meta-space-time product search result archive indicator (a function of outputting the list of products corresponding to the result value through the meta-space-time product identification information transmission module on the screen of the product coordinate terminal, etc.), etc.
- The meta-space-time product access interface may be configured to include
   -- a meta-space-time product access module (meaning the URL or IP address, or a means such as the blockchain or NFT; a function of accessing the web page, the database, or the transaction data block, etc., through the corresponding implemented means, etc.) etc.

As an example, there is a painter who has 3 million followers and paints pictures of the future. This painter has uploaded many his/her paintings based on 0:00 on December 24th, 2030, on social media.

When this painter tries to resell the shoes online, the most advantageous search keyword is not shoes, but 0:00 on December 24th, 2030.

Even if the purpose is to sell the shoes, but the actual shoes and this date are completely unrelated, due to the nature of the Internet, pages with high user traffic are more advantageous for selling products or services compared to pages with low user traffic.

According to the present invention, in such cases, as the search, advertisement, and distribution markets change due to problems with personal information collection, etc., the corresponding shoes may be searched for through a search centered on the space-time coordinates into which the space coordinates (latitude and longitude that are 1D location information of the shoes displayed on the store shelf, and 3D height information) of the products displayed in the shoe store are integrated and the time coordinates of 0:00 on December 24th, 2030, are integrated, and the shoes may be traded by netizens through the search.

As another example, high school student Kim Cheol-su tries to identify products in an offline store by capturing the products with a camera to resell the products. In this case, when the products are captured, the artificial intelligence immediately identifies what kind of product it is in real time, and the identified product is assigned the integrated space-time coordinates in real time, and the search (or access) and trading for the products may be performed.

As another example,
Middle school student Kim Young-hee tries to resell products (or services) that are being sold online. However, the existing technology may search for and resell products or services into which the space-time coordinates are integrated only in the environment where the products or services mainly exist on the Internet, and may not search for and resell products for which this is not the case. However, according to the present invention, in such cases, the coordinates based on the latitude and longitude, and the height information of the actual products (or services) are identified in real time, and the space-time coordinates in which the certain point-in-time (or period) information is integrated are assigned to the identified coordinates, and netizens can search for (or access) and trade the products or services, and as a result, middle school student Kim Young-hee may share a certain amount of revenue with the original seller of the product.

Meanwhile, according to the present invention, in addition to the case where the artificial intelligence identifies images captured with a camera, etc., to identify products (or services), the space-time coordinates are integrated and assigned in real time to products (or services) that are identified online or offline through the VR, the AR, etc., using desk top VR, projected VR, immerse VR using a head mounted display (HMD), a computer assisted virtual environment (CAVE), and other devices (smart glasses, a device for detecting Braille or pressure, a device for detecting sound, a device for detecting smell, etc.), and the search (or access) and trade for the products or services can be performed.

## Claims

1. A meta-space-time-based product trading device that transcends time and space, comprising:
a meta-space-time product coordinate generator that generates meta-space-time product coordinate information in which coordinates corresponding to specific latitude information, longitude information, and height information for a specific product are integrated into a single set of coordinates, and assigns the generated meta-space-time product coordinate information to the specific product; and
a resale product information database that is linked to the meta-space-time product coordinate generator and in which information about the product assigned the meta-space-time product coordinate information is recorded,
wherein the meta-space-time-based product trading device further includes a purchase interface that is linked to a resale product information database to trade the meta-space-time product coordinate information or receive, via the Internet, data for trading the product assigned the meta-space-time product coordinate information.

2. The meta-space-time-based product trading device of claim 1, further comprising a means that generates the meta-space-time product coordinate information in which coordinates corresponding to the specific latitude information, longitude information, and height information, and specific point-in-time information or specific period information are integrated into the single set of coordinates, and assigns the generated meta-space-time product coordinate information to the specific product.

3. The meta-space-time-based product trading device of claim 1, wherein the resale product information database further includes a function of storing information for trading the product assigned the meta-space-time product coordinate information in which the coordinates corresponding to the specific latitude information, longitude information, and height information for the specific product are integrated into the single set of coordinates, or generating and storing a transaction data block that is information on trading the meta-space-time product coordinate information.

4. The meta-space-time-based product trading device of claim 3, wherein the transaction data block further includes hash information for identifying or accessing the specific latitude information, longitude information, and height information for the specific product, and the specific point-in-time information or the specific period information.

5. The meta-space-time-based product trading device of claim 3, wherein the transaction data block further includes hash information for identifying or accessing at least one of the sound information, smell information, pressure size information, or image information for the specific product.

6. The meta-space-time-based product trading device of claim 3, wherein the transaction data block is composed of a non-fungible token (NFT).

7. The meta-space-time-based product trading device of claim 1 or 3, wherein the resale product information database further includes a database for accessing and retrieving at least one of sound information, smell information, pressure size information, and image information that identify a specific product via the Internet.

8. The meta-space-time-based product trading device of claim 1, wherein the meta-space-time product coordinate generator further includes:
a product location coordinate setting module that inputs the specific latitude information and longitude information for the specific product;
a product height coordinate setting module that inputs specific height information; and
a means that generates the meta-space-time product coordinate information in which the information input from each of the product location coordinate setting module and the product height coordinate setting module is integrated into the single set of coordinates and assigning the generated meta-space-time product coordinate information to the specific product.

9. The meta-space-time-based product trading device of claim 1, wherein the meta-space-time product coordinate generator further includes a means that integrates information input from either a product point-in-time coordinate setting module inputting specific point-in-time information or a product period coordinate setting module inputting specific period information into the meta-space-time product coordinate information and assigns the information to the specific product.

10. The meta-space-time-based product trading device of claim 1, wherein the meta-space-time product coordinate generator further includes a product location information identification module that identifies the latitude information and the longitude information where the specific product is located, a product height information identification module that identifies the meta-space-time product coordinate information in which the information identified from the product location information identification module and the product height information identification module is integrated into the single set of coordinates and assigns the identified meta-space-time product coordinate information to the specific product.

11. The meta-space-time-based product trading device of claim 1, wherein the meta-space-time product coordinate generator further includes a means that further integrates information identified from either a product point-in-time information identification module identifying specific point-in-time information or a product period information identification module identifying the specific period information into the meta-space-time product coordinate information and assigns the information to the specific product.

12. The meta-space-time-based product trading device of any one of claims 8 to 11, wherein the meta-space-time product coordinate generator further includes a barcode identification module that identifies a barcode for identifying the specific product.

13. The meta-space-time-based product trading device of any one of claims 8 to 11, wherein the meta-space-time product coordinate generator further includes a sound detection module that identifies a sound for identifying the specific product.

14. The meta-space-time-based product trading device of any one of claims 8 to 11, wherein the meta-space-time product coordinate generator further includes a smell detection module that identifies a smell for identifying the specific product.

15. The meta-space-time-based product trading device of any one of claims 8 to 11, wherein the meta-space-time product coordinate generator further includes a smell detection module that identifies certain pressure size information to identify the specific product.

16. The meta-space-time-based product trading device of any one of claims 8 to 11, wherein the meta-space-time product coordinate generator further includes an image information identification module that identifies a product corresponding to image information for a product recognized by a camera connected via the Internet to identify the specific product.

17. The meta-space-time-based product trading device of any one of claims 8 to 11, wherein the meta-space-time product coordinate generator further includes an image information identification module that identifies a product corresponding to an image or a video of the specific product to identify the specific product.

18. The meta-space-time-based product trading device of any one of claims 8 to 11, wherein the meta-space-time product coordinate generator further includes a specific code information input module that identifies a product corresponding to specific code information to identify the specific product.

19. The meta-space-time-based product trading device of claim 1, wherein the meta-space-time product trading device is linked to a database accessed via the Internet, and further includes an artificial intelligence identification module that identifies a product corresponding to any one of sound information, smell information, pressure size information, or image information identified through any one of a sound detection module, a smell detection module, a pressure detection module, or an image information identification module, using artificial intelligence.

20. The meta-space-time-based product trading device of claim 1, wherein the meta-space-time product coordinate information is composed of a specific uniform resource locator (URL).

21. The meta-space-time-based product trading device of claim 1, wherein the meta-space-time product coordinate information is composed of a specific Internet protocol (IP) address.

22. The meta-space-time-based product trading device of claim 1, wherein the meta-space-time product coordinate information is composed of a specific source code.

23. The meta-space-time-based product trading device of claim 1, wherein the meta-space-time product coordinate information is configured to further include information that identifies a floor of a specific building where the specific product is located.

24. The meta-space-time-based product trading device of claim 8, wherein the product height coordinate setting module is configured to further include a means that inputs information that sets a floor of a specific building where the specific product is located.

25. The meta-space-time-based product trading device of claim 8, wherein the product height coordinate setting module is configured to further include a means that inputs or identifies information that sets or identifies a height of a specific product from sea level or the ground.

26. The meta-space-time-based product trading device of claim 1, further comprising a settlement management module that generates and transmits command information that instructs transfer of an amount according to a commission amount or commission rate information for a sale of the specific product to a reseller's account.

27. The meta-space-time-based product trading device of any one of claims 8 to 11, wherein a screen of the meta-space-time product coordinate generator is a display device constituting a head mounted display (HMD).

28. The meta-space-time-based product trading device of any one of claims 8 to 11, wherein a screen of the meta-space-time product coordinate generator is a display device constituting smart glasses.

29. The meta-space-time-based product trading device of any one of claims 8 to 11, wherein a screen of the meta-space-time product coordinate generator is a display device that outputs image information recognized by a camera connected via the Internet and data retrieved via the Internet together.

30. The meta-space-time-based product trading device of claim 27, further comprising a database for retrieving data output by the display device constituting the EA/LD via the Internet.

31. The meta-space-time-based product trading device of claim 28, further comprising a database for retrieving data output by the display device constituting the smart glasses via the Internet.

32. The meta-space-time-based product trading device of claim 29, further comprising a database for, when the image information recognized by the camera and the data retrieved via the Internet are output to the display device together, retrieving the corresponding data via the Internet.

33. A meta-space-time product search and access device that transcends time and space, wherein the meta-space-time product search and access device is linked to a resale product information database, and retrieves list information of a product assigned meta-space-time product coordinate information in which coordinates corresponding to specific latitude information, longitude information, and height information for a specific product are integrated into a single set of coordinates from data stored in the resale product information database, and outputs the information on a screen of a meta-space-time product coordinate generation terminal.

34. The meta-space-time product search and access device of claim 33, further comprising a meta-space-time product radius information input module that inputs range information for a radius from the meta-space-time product coordinate information.

35. The meta-space-time product search and access device of claim 34, wherein the meta-space-time product radius information input module further includes a means that inputs point-in-time information or period information for a specific product.

36. The meta-space-time product search and access device of claim 33, wherein the meta-space-time product search and access device further includes a meta-space-time product access module that accesses a specific web page or accesses data of the transaction data block through a URL, an IP address, or a source code.

37. A meta-space-time product trading method of trading a product assigned meta-space-time coordinates online, comprising:
inputting at least one of information of latitude and longitude, height, point in time, and period of a product to be searched for to input information for searching for a product assigned resale product information through a meta-space-time product information input module;
outputting a list of products assigned the resale product information on a screen of a product coordinate terminal through a meta-space-time product search result archive indicator;
accessing a meta-space-time product access module to access comprehensive product information of one of the products among the list of products output on the screen of the product coordinate terminal;
executing a function of purchasing the product assigned the resale product information accessed through the meta-space-time product access module; and
storing trading-related information for a resale product in a trading information log database.

38. A meta-space-time product coordinate generation method using a selection method of selecting a product or service through a product selection module and generating meta-space-time product coordinates and assigning the generated meta-space-time product coordinates to the selected product or service, comprising:
logging in to each account login module of at least one of a reseller account, an original seller account, and a buyer account;
selecting the product through the product selection module;
outputting a store list of products selected through the product selection module through a store archive indicator;
identifying or setting at least one of information on a point in time and a period from at least one of a product point-in-time coordinate setting module and a product period coordinate setting module;
approving resale of a product to which resale product information is assigned by a resale approval module according to a schedule protocol;
assigning at least one of information on latitude and longitude, height, point in time, and period identified or set by at least one of a product location coordinate setting module, a product height coordinate setting module, a product point-in-time coordinate setting module, and a product period coordinate setting module to the comprehensive product information stored in a product information storage device; and
generating resale product information for assigning resale product information to the comprehensive product information stored in a product information storage device, and assigning the generated resale product information to the corresponding comprehensive product information.

39. A meta-space-time product coordinate generation method using an identification or detection method of identifying or detecting a product or service through a schedule identification or detection means installed in a product coordinate terminal, generating meta-space-time product coordinates, and assigning the generated meta-space-time coordinates to the identified product or service, comprising:
logging in to each account login module of at least one of a reseller account, an original seller account, and a buyer account;
identifying or detecting a product by at least one of a product identification interface image information identification module, a virtual reality (VR) identification module, an augmented reality (AR) identification module, a barcode identification module, a sound detection module, a smell detection module, a pressure detection module, and a product identification code information input module of a product identification interface;
outputting a store list of products identified (or detected) by the product identification interface or a store list identified by a store location information identification module through a store archive indicator;
selecting at least one store from among stores in the store list output from the store archive indicator;
identifying at least one of latitude and longitude and height information from at least one of a product location coordinate setting module and a product height coordinate setting module;
identifying or setting at least one of information on a point in time and a period from at least one of a product point-in-time coordinate setting module and a product period coordinate setting module;
approving resale of a product to which resale product information is assigned by a resale approval module according to a schedule protocol;
assigning at least one of information on latitude and longitude, height, point in time, and period identified or set by at least one of a product location coordinate setting module, a product height coordinate setting module, a product point-in-time coordinate setting module, and a product period coordinate setting module to the comprehensive product information stored in a product information storage device; and
generating resale product information for recording resale product information in a resale product information database synchronized with comprehensive product information stored in the product information storage device, and recording the generated resale product information in the database.
